# EUROPEAN PATENT SPECIFICATION

(11) **EP 4 114 521 B1**
(45) Date of publication and mention of the grant of the patent: **06.05.2026**
(21) Application number: 21711189.7
(22) Date of filing: 05.03.2021
(51) Int. Cl.: A61P 5/00, A61P 25/00, A61Q 17/04, A61Q 19/08, A61K 8/9789, A61K 36/744, A61K 8/34, A61K 8/44, A61K 8/60

(54) **COSMETIC COMPOSITION**
KOSMETISCHE ZUBEREITUNG
COMPOSITION COSMÉTIQUE

(30) Priority: 05.03.2020 GB 202003184
(43) Date of publication of application: 11.01.2023
(73) Proprietor: Givaudan SA, 1214 Vernier (CH)
(72) Inventor: SENNELIER PORTET, Bénédicte, 84450 Jonquerettes (FR); REYNAUD, Romain, 31400 Toulouse (FR); SCANDOLERA, Amandine, 08190 Sault-Saint-Remy (FR); DE TOLLENAERE, Morgane, 51100 Reims (FR)
(74) Representative: Global Patents
(86) International application number: PCT/EP2021/055532
(87) International publication number: WO 2021/176026

(56) References cited:
- EP-A1- 3 280 276
- WO-A1-2010/061574
- WO-A1-2018/162760
- KR-A- 20100 038 797
- US-A1- 2014 141 082
- AICHA LAABICH ET AL: "Protective Effect of Crocin against Blue Light- and White Light-Mediated Photoreceptor Cell Death in Bovine and Primate Retinal Primary Cell Culture", INVESTIGATIVE OPTHALMOLOGY & VISUAL SCIENCE, vol. 47, no. 7, 1 July 2006 (2006-07-01), US, pages 3156, XP055235898, ISSN: 1552-5783, DOI: 10.1167/iovs.05-1621

## Description

The present invention relates to cosmetic compositions comprising a Gardenia fruit extract.

In modern society, digital technology is becoming ever more common, leading to an increased exposure to blue light.

Blue light is a type of high-energy light and is part of the visible light spectrum, having a wavelength between 400-495 nm. Typical blue light sources include mobile phones, computers, tablets, televisions, and lights. Much of the exposure arises from light emitting diodes (LEDs).

Increasingly, people are exposed to blue light via everyday technology. The 2015 Pew Research Center study found that 68% of U.S. adults own a smartphone and 45% own a tablet. The study also found that levels of technology ownership vary by age; 86% of Americans 18-29 and 83% of those 30-49 own smartphones. In contrast, computer ownership rates are lower for older Americans.

Blue light seems to affect the skin and can damage cells. Moreover, Dong et al. demonstrated that blue light disrupts the circadian rhythm, affecting people's sleep quality.

Melatonin is a well-known sleep-related hormone naturally secreted by our organism (brain and skin), on a daily cycle. Its production peak happens at night and plays a crucial role in our capacity to fall asleep and in our sleep quality. In addition, melatonin also acts as a powerful anti-ageing agent, thanks to its strong antioxidant properties, and the triggering of a biological defenses cascade through its binding to the melatonin membrane receptor MT1R.

Exposure to blue light leads to a disturbance of the melatonin production rhythm, causing skin ageing (loss of antioxidant defences and damages to the mitochondria) and a perturbation of the sleep-related functions (difficulties to fall asleep, waking-up several times at night, fatigue in the morning). This results in a premature ageing of the skin, which is more exposed to external aggressions and unable to restore itself during resting phases of the human body.

Korean Patent application KR-A2010/0038797 discloses skin ageing compositions containing a complex crude drug of the Gardeniae Fructus and Coptidis Rhizoma, the Scutellariae Radix, the Phellodendri Cortex, the Forsythiae Fructus, the Lonicerae Flos as active ingredient with betain, glycerol, water and butylenglycol as solvents.

US Patent application US-A-2014/141082 discloses compositions comprising at least one of crocin and crocetin, which are extracted, enriched and/or purified from natural source such as fruits of gardenia.

It is therefore an objective of the present invention to provide a cosmetic means for preventing the negative effects caused by the exposure to blue light.

This problem has been solved by the cosmetic active agent and cosmetic composition of the present invention.

In a first aspect, the present invention provides a cosmetic active agent comprising a Gardenia fruit extract.

The Gardenia plant, which is also called cape jasmine or danh-danh, is an evergreen flowering plant of the coffee family *Rubiaceae.* It originated in Asia and is most commonly found growing wild in Vietnam, Southern China, Korea, Taiwan, Japan, Myanmar, India and Bangladesh. There are around 250 kinds of Gardenia in the world.

Throughout the present disclosure, the term "Gardenia" is meant to cover all species of the genus *Gardenia,* including *Gardenia jasminoides, Gardenia angustifolia, Gardenia augusta, Gardenia florida, Gardenia grandiflora, Gardenia radicans, Gardenia longisepala, Gardenia maruba,* and *Gardenia pictorum,* and in particular *Gardenia jasminoides* J. Ellis.

Traditionally, the Gardenia fruit has been used in oral applications of folk medicine for treating inflammation, headache, edema, fever, hepatic disorders, and hypertension. The fragrant flowers are also used for scenting tea, and are sometimes even eaten raw as a delicacy, pickled or preserved in honey. Nowadays, Gardenia plants are mainly exploited for food additives, dyestuffs, and as ornamental plant.

Gardenia fruits have a red color due to natural carotenoid pigments. In particular, they contain crocetin, crocin, and other crocetin esters.

The term "Crocins" as used throughout this disclosure, is meant to encompass the aglycon crocetin, its di-gentiobioside derivative crocin, as well as other crocetin mono- or diglucosides and any other crocetin derivatives having the 8,8'-diapocarotenedioic acid core.

Crocetin is a natural apocarotenoid dicarboxylic acid that is found in the *Crocus sativus L.* flower and *Gardenia jasminoides* fruits. It has the following structure:

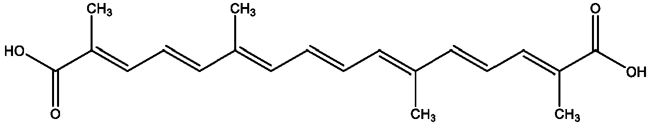

Crocin is the diester formed from the disaccharide gentiobiose and the dicarboxylic acid crocetin. It has the following structure:

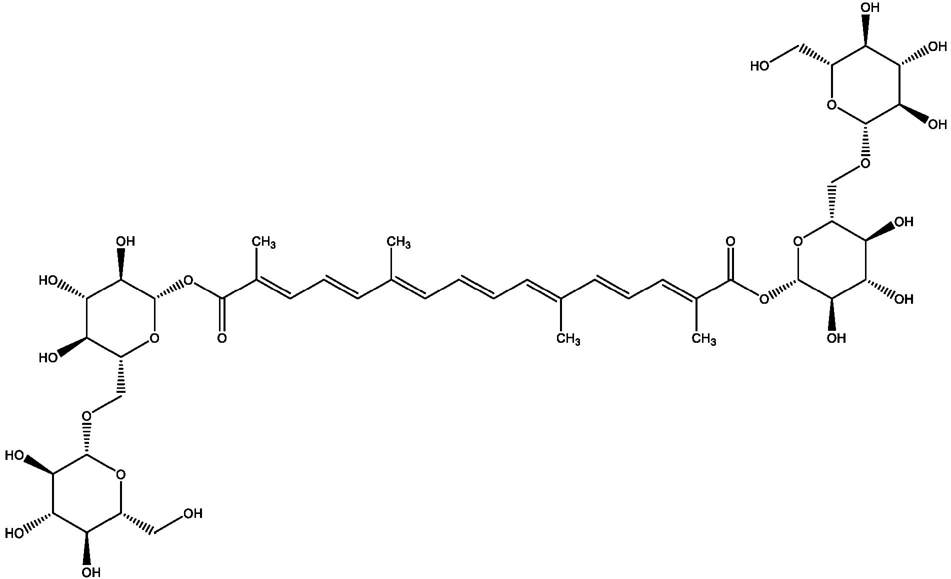

Crocin is one of the main components in Gardenia fruit extract.

Crocins are particularly interesting as they are among the few carotenoids soluble in water, which makes them suitable as a coloring agent for food applications.

One of the key disadvantages of the Crocins is their notorious instability, in particular due to degradation under the influence of light, decreasing pH and increasing temperature. This fact renders the cosmetic use of Gardenia fruit extract, and Crocins in general, difficult if not impossible.

Therefore, it is important to stabilize the Gardenia fruit extract within a cosmetic active agent.

Consequently, the cosmetic active agent of the present invention further comprises a solvent, which is able to stabilize the extract, and in particular the Crocins contained therein.

Surprisingly, it has been found that the stability of Crocins can be improved by the addition of a eutectic solvent having a pH of at least 5. This finding has been confirmed by long-term stability studies (see Example 2 below).

Therefore, the solvent used in the cosmetic active agent of the present invention is a eutectic solvent having a pH of at least 5. The eutectic solvent allows for a good solubilization of the Gardenia fruit extract, and also for its stabilization, in particular of the Crocins contained therein.

Eutectic solvents are generally known. They consist of several components, typically at a specific concentration range each, and possess a lower melting point than the individual components.

The cosmetic active agent of the present invention is for topical application.

The cosmetic active agent of the present invention has been found to have several advantageous effects, which have been proven both by *in vitro* and clinical studies (see examples below.

In particular, it was found to have anti-ageing properties and to improve the sleep quality, in particular of people with high exposure to blue light.

Without being bound by theory, it is believed that the cosmetic active agent of the present invention protects the cutaneous production of melatonin when skin is exposed to digital stress, i.e. blue light, thereby preventing premature skin ageing.

It has been found that the crocin present in the cosmetic active agent of the present invention not only protects the natural skin melatonin cycle, but can also be converted to crocetin by the skin microflora. Based on a microbiome study (see example 3 below), crocin is completely transformed to crocetin within about 210 hours in culture.

Without being bound by theory, applicant believes that crocetin is able to bind to the main melatonin receptor MT1R, whereas crocin does not. This hypothesis is supported by molecular modelling studies based on the crystallographic structures of MT1R, crocin, crocetin and melatonin, which were retrieved from the Protein Data Bank (www.rcsb.org). A calculation of the molecular interaction for each of the other molecules with MT1R showed that crocetin has a positive affinity score of 3.6232, which is very close to that of melatonin (5.6553), while crocin has a negative affinity score of -36.4141.

Thus, it is believed that crocetin is able to trigger the skin melatonin receptors (MT1). Therefore, thanks to its activation by the skin microbiome, the cosmetic active agent of the present invention can take an active part in skin defense and repair mechanisms.

Clinical tests (see example 8 below) have shown that the cosmetic active agent of the present invention reverses visible signs of ageing (wrinkle number -21% versus placebo). It is therefore perfectly suitable for preventing and curing premature skin ageing. This finding was also confirmed by a self-assessment of the panelists, during which 80% of the volunteers stated that their skin was more hydrated (40% for placebo) and 75% of the volunteers stated that their skin was smoother (45% for placebo).

More specifically, the cosmetic active agent of the present invention is able to protect the mitochondrial network and cell spreading *in vitro* (see examples 5 and 6 below). It was shown that exposure to blue light damages the mitochondrial network. In the presence of the cosmetic active agent of the present invention, however, the network is less fragmented. Cell spreading is also significantly affected by blue light, but again, the cosmetic active agent of the present invention is able to prevent this effect.

The cosmetic active agent of the present invention is also able to reduction the oxidized protein content *ex vivo* (see example 7 below). After exposure to blue light, the oxidized protein content in human skin explants is significantly increased. This effect can be reversed by the cosmetic active agent of the present invention.

Furthermore, the cosmetic active agent of the present invention was found to preserve the melatonin cycle *in vitro* (see example 4 below), ensuring the melatonin release during the night and thus protect skin against digital stress.

The cosmetic active agent of the present invention has further been found to have a positive effect on sleep quality, thereby contributing to the overall well-being.

In particular, a topical application of the cosmetic active agent has been found to reduce the number of awakenings during the night and to increase the easiness to fall asleep (see clinical studies in example 8 below). It is believed that the cosmetic active agent acts as a vegetal melatonin-like molecule, activating biological mechanisms connected to the circadian rhythm.

In an embodiment of the present invention, the eutectic solvent has a pH of at least 5.5, more preferably of at least 6, and most preferably of at least 7. Thanks to the higher pH, the stabilization of the Crocins in the Gardenia fruit extract is improved. However, for skin care applications, it is desirable that the pH is not too high, e.g. not higher than 10, more preferably not higher than 9, and most preferably not higher than 8.

In an embodiment of the present invention, the components of the eutectic solvent are of natural origin. Eutectic solvents formed of components of natural origin are generally known and are typically described as "natural deep eutectic solvents", or NaDES.

More preferably, the components of the eutectic solvent are all of 100% natural origin according to ISO16128.

In an embodiment of the present invention, the eutectic solvent comprises betaine, glycerol and water. It has been found that this eutectic solvent is particularly well suited for the solubilization and stabilization of the Gardenia fruit extract. Also, betaine can act as a moisturizing agent in skin care applications.

Eutectic solvents based on glycerol, betaine and water are generally known (e.g. from WO 2016/162703). Apart from avoiding the drawbacks of common synthetic organic solvents - such as inherent toxicity, high volatility, or lack of renewability - they also have the advantage of being of natural origin. For this reason, they are also described as "natural deep eutectic solvents", or NaDES.

In an embodiment of the present invention, the eutectic solvent comprises from about 30 to about 40 wt% of betaine, from about 35 to about 45 wt% of glycerol, and from about 20 to about 30 wt% of water, more preferably about 35 wt% of betaine, about 40 wt% of glycerol, and about 25 wt% of water.

In a particularly preferred embodiment, the eutectic solvent consists of 34.6 wt% of betaine, 40.4 wt% of glycerol, and 25.0 wt% of water. This eutectic solvent has a pH of about 7.45.

Without being bound by theory, applicant believes that, in the presence of a eutectic solvent based on betaine, glycerol and water, a transesterification takes place whereby at least one of the gentiobioside moieties of crocin is replaced by a glycerol-betaine moiety to form the following adduct:

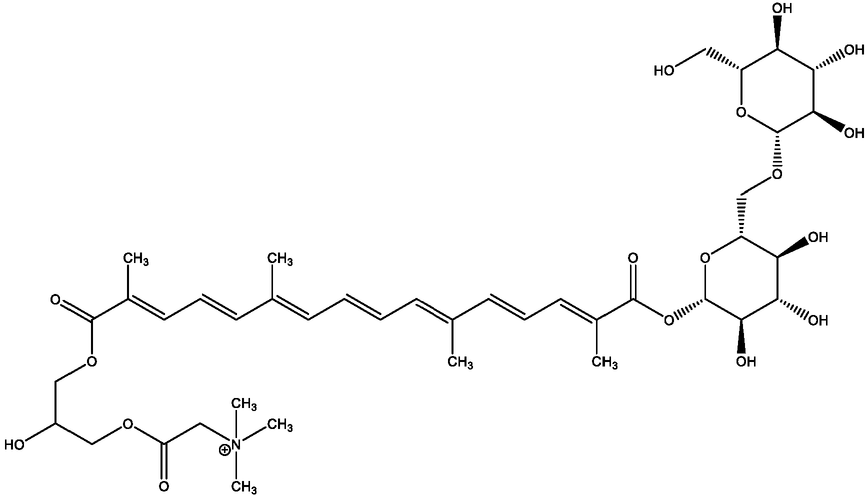

This hypothesis has been supported by 2D-NMR analysis (NOESY and HSQC).

This derivative is also meant to be included in the term "Crocins" as defined above.

In an alternative embodiment, the eutectic solvent is a mixture of pentylene glycol, betaine and water. In particular, the eutectic solvent may comprise from about 35 to about 50 wt% of pentylene glycol, from about 25 to about 35 wt% of betaine, and from about 20 to about 35 wt% of water, more preferably about 44 wt% of pentylene glycol, about 29 wt% of betaine, and about 28 wt% of water. In a particularly preferred embodiment, the eutectic solvent consists of 43.7 wt% of pentylene glycol, 28.8 wt% of betaine, and 27.5 wt% of water. This eutectic solvent has a pH of about 6.85.

The concentration of the Gardenia fruit extract in the cosmetic active agent of the present invention should be chosen such that the above described advantageous effects can be achieved.

In an embodiment of the present invention, the concentration of the Gardenia fruit extract in the cosmetic active agent is from about 0.01 to about 10 wt%, more preferably from about 0.05 to about 1 wt%, and most preferably about 0.1 wt%.

To ensure the activity of the cosmetic active agent of the present invention, a relatively high content in Crocins in the Gardenia fruit extract is preferred. As can be seen from the analysis of commercially available Gardenia fruit extracts (see example 1 below), the Crocin content can vary from one supplier to another.

In an embodiment of the present invention, the Gardenia fruit extract comprises at least 1 wt% of Crocins, more preferably at least 10 wt% of Crocins, and most preferably at least 25 wt% of Crocins.

In a further aspect, the present invention provides a cosmetic composition comprising the cosmetic active agent of the present invention and a cosmetically acceptable excipient. The cosmetic composition of the present invention is meant for topical application.

Any excipients commonly used in the preparation of cosmetic preparations for use on the human skin may be employed in the present invention. Suitable excipients include, but are not limited to ingredients that can influence organoleptic properties, penetration of the skin, and the bioavailability of the Gardenia fruit extract. More specifically, they include liquids, such as water, oils or surfactants, including those of petroleum, animal, plant or synthetic origin, such as and not restricted to, peanut oil, soybean oil, mineral oil, sesame oil, castor oil, polysorbates, sorbitan esters, ether sulfates, sulfates, betaines, glycosides, maltosides, fatty alcohols, nonoxynols, poloxamers, polyoxyethylenes, polyethylene glycols, dextrose, glycerol, digitonin, and the like.

The formulation for topical application to the skin may take any physical form. For instance, the cosmetic composition, and in particular the skin care composition, may be in the form of a liposome composition, mixed liposomes, oleosomes, niosomes, ethosomes, milliparticles, microparticles, nanoparticles and solid-lipid nanoparticles, vesicles, micelles, mixed micelles of surfactants, surfactant-phospholipid mixed micelles, millispheres, microspheres and nanospheres, lipospheres, millicapsules, microcapsules and nanocapsules, as well as microemulsions and nanoemulsions, which can be added to achieve a greater penetration of the Gardenia fruit extract.

The cosmetic composition, and in particular the skin care composition, may be produced in any solid, liquid, or semi-solid form useful for application to the skin topically or by transdermal application. Thus, these preparations of topical or transdermal application include, but are not restricted to, creams, multiple emulsions, such as and not restricted to, oil and/or silicone in water emulsions, water-in-oil and/or silicone emulsions, water/oil/water or water/silicone/water type emulsions, and oil/water/oil or silicone/water/silicone type emulsions, micro-emulsions, emulsions and/or solutions, liquid crystals, anhydrous compositions, aqueous dispersions, oils, milks, balsams, foams, aqueous or oily lotions, aqueous or oily gels, cream, hydro-alcoholic solutions, hydro-glycolic solutions, hydrogels, liniments, sera, soaps, face masks, serums, polysaccharide films, ointments, mousses, pomades, pastes, powders, bars, pencils and sprays or aerosols (sprays), including leave-on and rinse-off formulations.

Thus, the present invention also provides a skin care composition, and in particular an anti-ageing skin care composition.

The advantageous effects of the cosmetic active agent of the present invention, and thus also of the cosmetic composition of the present invention, have been described in detail above.

The specific embodiments of the cosmetic active agent are advantageously also applied to the cosmetic composition.

In a further aspect, the present invention also provides a method of reducing the signs of ageing in skin, comprising the step of topically applying the cosmetic active agent or the cosmetic composition of the present invention to the skin, in particular to facial skin. The advantageous effects have been described in detail above and are further supported by the examples below.

In a further aspect, the present invention also provides a method of protecting the skin against oxidative stress, comprising the step of topically applying the cosmetic active agent or the cosmetic composition of the present invention to the skin, in particular to facial skin. The advantageous effects have been described in detail above and are further supported by the examples below.

In a further aspect, the present invention also provides a method of protecting the skin against the effects of blue light, comprising the step of topically applying the cosmetic active agent or the cosmetic composition of the present invention to the skin, in particular to facial skin. The advantageous effects have been described in detail above and are further supported by the examples below.

In a further aspect, the present invention also provides a non-therapeutic method of protecting an individual's melatonin cycle, comprising the step of topically applying the cosmetic active agent or the cosmetic composition of the present invention to the skin, in particular to facial skin. The advantageous effects have been described in detail above and are further supported by the examples below.

In a further aspect, the present invention also provides a non-therapeutic of improving an individual's sleep, comprising the step of topically applying the cosmetic active agent or the cosmetic composition of the present invention to the skin, in particular to facial skin. The advantageous effects have been described in detail above and are further supported by the examples below.

In a further aspect, the present invention also relates to the use of a Gardenia fruit extract for improving an individual's sleep.

In particular, the present invention also relates to the use of the cosmetic active agent or the cosmetic composition of the present invention for improving an individual's sleep.

The present invention is further illustrated by means of the following non-limiting examples:

### Example 1: Gardenia Fruit Extract

Gardenia fruit extract is commercially available from several suppliers.

For the present studies, commercial extracts were obtained from Indfrag Bioscience Private Limited ("Gardenia Florida Extract", batch Nos. GFP-ROE-14002 and GFP-ROE-19001 ; plant cultivated in Anhui province in China; according to supplier specifications, the extract is obtained from the *Gardenia Florida* fruit by water extraction, fractionation, concentration, drying and powdering) and Yunnan Rainbow Bio-tech. Corp., Ltd ("Gardenia yellow powder", batch No. ZT180201, received on 6 July 2018; plant cultivated in Guangxi province in China; according to supplier specifications, the extract is obtained from the Gardenia fruit (*Gardenia Jasminoides* Ellis) by extraction, filtration, refining, concentration and spray drying). Both products are in powder form, and are yellowish to reddish or brownish.

The authenticity of the products was confirmed by macroscopic and microscopic identification, HPTLC, DNA analysis (external provider: Tru-ID), and HPLC of a dried fruit sample obtained from each supplier.

The Gardenia fruit extracts of both suppliers were further analyzed by HPLC-UV and LC-TOF to determine their composition. It was found that the sample from Indfraq contained about 10 wt% of Crocins, whereas that of Yunnan Rainbow contained about 30 wt%.

The commercial Gardenia fruit extracts were further processed as follows:
- grinding
- extraction with water (Yunnan Rainbow) or methanol/water 75:25 (Indfrag)
- filtration
- adsorption and desorption with ethanol (70%)
- concentration
- spray-drying with maltodextrin (for Yunnan Rainbow only)

The thus obtained solid material was then dissolved in the desired solvent(s).

### Example 2: Stability Studies

The stability of Gardenia fruit extract in different solvents was tested in different conditions.

### Long Term Stability

The long term stability was tested at room temperature and at 40 °C in two different eutectic solvents: (a) a mixture of betaine, lactic acid and water, and (b) a mixture of betaine, glycerol and water. The pH of the solvents alone is (a) 3.27 and (b) 7.45, respectively. Gardenia fruit extracts from both Yunnan Rainbow (a1) and (b1) and Indfrag (a2) and (b2) were tested.

The eutectic solvents were prepared by mixing the components at 50 °C. 0.1 wt% of Gardenia fruit extract (in powder form) was then added and the obtained composition was heated to 80 °C for 1 hour.

The samples were stored at room temperature and at 40 °C, respectively, for a period of 3 months. The stability was assessed by measuring the pH, the Gardener index and the optical density at λₘₐₓ (in 250-600 nm range). The results are presented in the following table:

| | Time | 0 | 1 month | | 2 months | | 3 months | |
|---|---|---|---|---|---|---|---|---|
| | Temperature | rt | rt | 40 °C | rt | 40 °C | rt | 40 °C |
| (a1) betaine / lactic acid / water (40:35:25 w/w) | pH | 3.78 | 3.53 | 3.19 | 3.63 | 3.33 | 3.65 | 3.22 |
| | Gardener index | 13 | 11 | 11 | 11 | 10 | 11 | 10 |
| | Optical density | 1.53 | 1.30 | 0.73 | 1.13 | 0.40 | 0.85 | 0.25 |
| (a2) betaine / lactic acid / water (40:35:25 w/w) | pH | 3.28 | 3.05 | 3.08 | 3.08 | 3.13 | 3.14 | 3.06 |
| | Gardener index | 11 | 11 | 10 | 10 | 10 | 10 | 10 |
| | Optical density | 0.50 | 0.38 | 0.20 | 0.38 | 0.1 | 0.35 | 0.11 |
| (b1) betaine / glycerol / water (40.4:34.6:25 w/w) | pH | 7.56 | 5.97 | 7.01 | 6.13 | 6.43 | 6.37 | 6.34 |
| | Gardener index | 13 | 12 | 11 | 12 | 11 | 12 | 11 |
| | Optical density | 1.69 | 1.97 | 1.18 | 1.80 | 1.00 | 1.51 | 0.80 |
| (b2) betaine / glycerol / water (40.4:34.6:25 w/w) | pH | 7.13 | 5.55 | 7.02 | 5.79 | 6.56 | 5.95 | 6.25 |
| | Gardener index | 11 | 11 | 10 | 11 | 10 | 10 | 10 |
| | Optical density | 0.54 | 0.56 | 0.38 | 0.46 | 0.36 | 0.44 | 0.28 |

As can been seen from the above, the Gardenia fruit extract was clearly more stable in the betaine / glycerol / water mixture compared to the betaine / lactic acid / water mixture.

### Samples for Sunlight ("Suntest") and Elevated Temperature Studies

Gardenia fruit extract (in powder form; from Yunnan Rainbow) was dissolved in (c) water and (d) a eutectic solvent formed from betaine, glycerol and water (35:40:25 w/w), respectively.

The eutectic solvent (d) was prepared by mixing the components at 50 °C until completely homogeneous.

For each of the solvents, 0.1 wt% of Gardenia fruit extract was added and the obtained composition was heated to 80 °C for 1 hour.

At T0, the color of the samples was identified according to CIELAB:

| | L | a* | b* | C | h* |
|---|---|---|---|---|---|
| (c) water | 67.26 | 52.94 | 114.73 | 126.35 | 65.30 |
| (d) betaine / glycerol / water (35:40:25 w/w) | 70.56 | 49.53 | 120.15 | 129.96 | 67.60 |

### Sunlight ("Suntest")

The samples were irradiated with 450 W/m² (300-800 nm) over a period of 24 hours. After 8, 12, 18 and 24 hours, the stability of the samples was assessed by measuring the pH, the change in color (dE2000), the Gardener index and the optical density at 440 nm (λₘₐₓ). The results are presented in the following table:

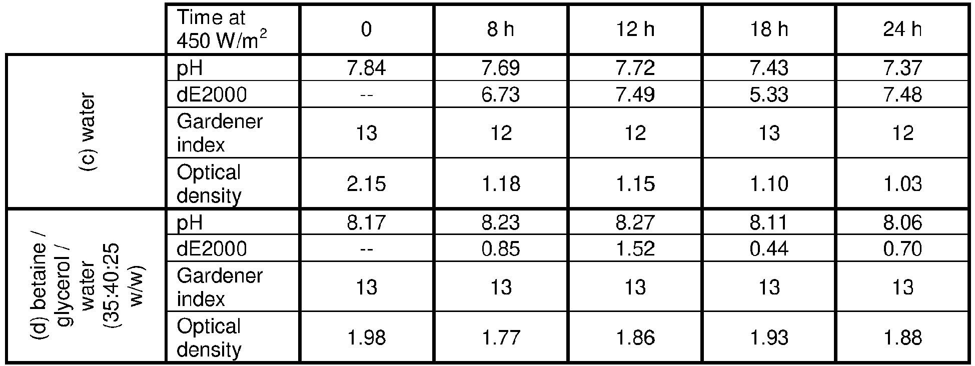

As can be seen from the above, the Gardenia fruit extract is perfectly stable in the eutectic solvent formed of betaine / glycerol / water, whereas the optical density decreases for the sample with only water, coupled with a significant change in color.

### Elevated Temperature

The samples were heated to 100 °C and 120 °C, respectively, over a period of 24 hours. After 8, 12, 18 and 24 hours, the stability of the samples was assessed by measuring the pH, the change in color (dE2000), the Gardener index and the optical density at 440 nm (λₘₐₓ). The results are presented in the following table:

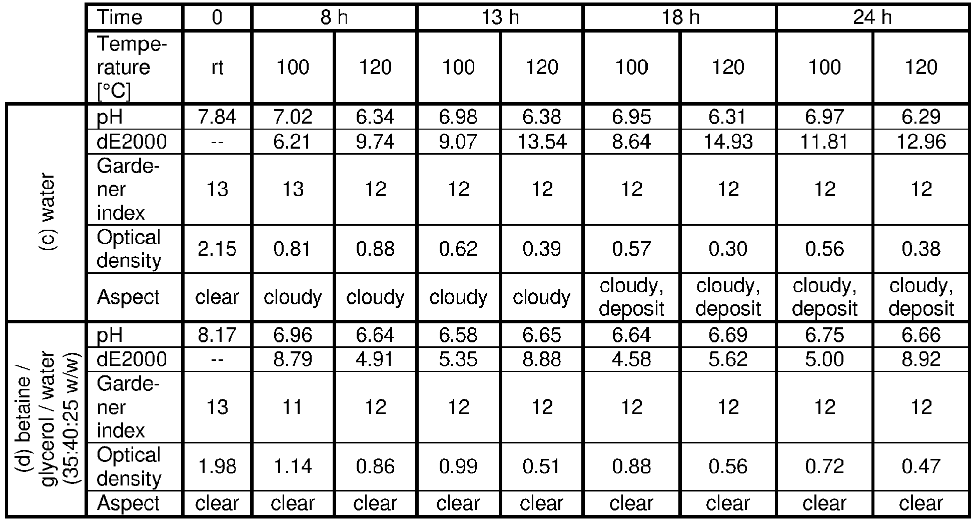

Again, the samples in water were less stable than those in the eutectic solvent consisting of betaine, glycerol and water. In particular, the samples in water became cloudy, and there was even a deposit formation at the later time points. For both solvents, the color changed visibly over time, but the change was more pronounced for the samples in water.

### Example 3: Conversion of Crocin to Crocetin by the Skin Microbiome (in vitro)

Sampling of skin microbiome was performed on 7 volunteers with a sterile gauze impregnated with NaCl in 5 areas: forehead, cheek, nose, neck, forearm. All samples were combined to obtain a representative skin microbiota composition.

The thus obtained microbiome was cultured in liquid medium (buffered HT medium) at 30 °C in the presence of crocin. Supernatants were collected regularly and analyzed by HPLC-MS to detect crocin and crocetin.

The results are shown in the following table:

| Time | 0 h | 18 h | 42 h | 66 h | 90 h | 162 h | 186 h | 210 h |
|---|---|---|---|---|---|---|---|---|
| Crocin concentration* | 100% | 100% | 47.5% | 2.9% | 0.1% | -- | -- | -- |
| Crocetin concentration* | -- | -- | 0.7% | 7.9% | 30.5% | 65.3% | 101.3% | 101.3% |

| | | | | | | | | |
|---|---|---|---|---|---|---|---|---|
| * % of maximum concentration detected | | | | | | | | |

As can be seen from the above, the microbiome present on the skin surface converts crocin to crocetin within a few days.

### Example 4: Evaluation of Melatonin Release (in vitro)

The influence of blue light on melatonin release was tested in an *in vitro* co-culture model of human sensory neurons and primary keratinocytes.

### Cell Culture

Sensory neurons were derived from hiPS (human induced Pluripotent Stem cells) cells themselves obtained from human fibroblasts. The hiPS were seeded into 6-well plates coated with a thin layer of Matrigel^{®} (Corning, ref: 354277, batch: 72005017) at a density of 250,000 cells per well in a differentiation medium consisting of DMEM-F12 (Panbiotech, ref: P04-41450, batch: 2730618) supplemented with 10% Knockout Serum Replacement (KSR, Life Technologies, ref: 10828028, batch: 1896527), 0.1µM of retinoic acid (Sigma, ref: R4643, batch: SLBF3638V), a cocktail of central differentiation pathway inhibitors and 1% of Penicilin-Streptomycin antibiotics (PS, Panbiotech, ref: P06-07100, batch: 7631018). The cells were maintained for 6 days in culture at 37 °C and 5% CO₂. The culture medium was changed every 2 days.

After 6 days of culture, the cells were dissociated using Accutase (Sigma Aldrich ref: A6964, batch: SLBT9789V) for 10 minutes at 37 °C. The reaction was stopped by adding culture medium. The cell suspension was centrifuged for 5 min at 1200 rpm. The cell viability was determined by cell counting with trypan blue and the cells were seeded into a 24-well plates coated with a thin layer of Matrigel^{®} at the density of 100,000 cells per well in the same differentiation medium as used before.

After 9 days of culture, the medium was replaced by a maturation medium for sensory neurons. This maturation medium was composed by DMEM-F12 supplemented with 1% of N2 (Life Technologies, ref: 11520536, batch: 2004543), 10 ng/mL of BDNF (PanBiotech, ref: CB-1115002, batch: 051861), 10 ng/mL of GDNF (PeproTech, ref: 450-10, batch: H170806), 10 ng/mL of NT3 (PeproTech, ref: 450-03, batch: H171010), 10 ng/mL of NGF (Sigma, ref: N1408, batch: SLBW7063) and 1% of antibiotics PS. Cells were maintained in culture at 37 °C and 5% CO₂. The culture medium was changed every 2 to 3 days.

After 14 days of culture, keratinocytes were added to the plates above the differentiated hiPS cell layer.

The keratinocytes, which were derived from a skin explant from a 30 years-old donor, were previously amplified in keratinocyte growth medium (Lonza, ref: 192152, batch 723883) on a cycle, before being dissociated by trypsination and frozen. These keratinocytes were thawed, and cell viability was determined by cell counting. The keratinocytes were seeded at 30,000 cells per well in a culture medium consisting of 2/3 of medium for sensory neurons and 1/3 of growth medium for keratinocytes. Cells were maintained in culture at 37 °C and 5% CO₂. The culture medium was changed every 2 to 3 days.

A cyclization protocol based on the use of Glutamate and a rise in temperature ("day" phase) was developed. In addition, shocks with a medium containing 50% of FCS were performed in order to synchronize the cell cycles (Ramanathan et al., Monitoring Cell-autonomous Circadian Clock Rhythms of Gene Expression Using Luciferase Bioluminescence Reporters, Journal of Visualized Experiments, 2012, 67: p.1-9; Buhr et al., Temperature as a universal resetting cue for mammalian circadian oscillators, Science, 2010, 330(6002): 379-385; Balsalobre et al., A Serum Shock Induces Circadian Gene Expression in Mammalian Tissue Culture Cells, Cell, 1998, 93: 929-937).

From this day on, the cultures were placed 8 hours per day under conditions allowing mimicking a day phase (glutamate 10 nM and a temperature of 39.5 °C).

On day 15 of co-culture, a medium rich in FCS (50% FCS and 50% medium 2/3 maturation medium for sensory neurons and 1/3 growth medium for keratinocytes) was incubated in the presence of the cells during the 2 first hours of the "day" phase. This FCS shock was applied to all cells (placed in day/night alternation and non-alternating controls).

On day 17 of co-culture, another FCS shock was applied to the cells. Gardenia Fruit extract (from Indfrag) was diluted at 0.004% (w/v) in the culture medium and applied to the cells during the "day" phase. On the same day, 30 min before the "night" phase, the cells of the co-cultures were exposed to a blue light.

From this day on, 0.004% (w/v) of Gardenia fruit extract was incubated with each change of culture medium. The culture was maintained under the same conditions, and treated with blue night every day 30 min before the "night" phase.

For analysis, samples of culture supernatants were taken 30 min before the "night" phase, and 2 h, 5 h and 8 h after the shift to the "night" phase and stored at -80 °C. This procedure was performed on days 17, 18 (i.e. 24 h after the last FCS shock), and 19 (i.e. 48 h after the last FCS shock). These culture supernatant samples were thawed and an ELISA assay was performed to dose the amount of melatonin released (BlueGene, ref ABIN511419).

After 20 days of culture, the cells were washed once with PBS and an MTT test was performed to validate cell viability.

### Statistical Analysis

The results were statistically analyzed by Kruskal-Wallis ANOVA followed by Mann Whitney U non-parametric test. Significance of results is indicated as p<0.05 with *, p<0.01 with ** and p<0.001 with ***.

### Results

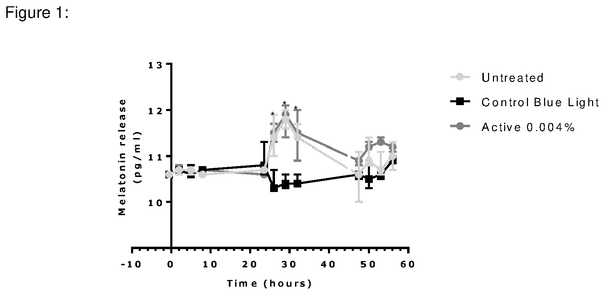

Figure 1 shows the melatonin release at different time points, with the x-axis indicating the time lapsed after the last FCS shock.

In the untreated control ("Untreated" in Figure 1), the synchronisation of the cells induced a cyclization of the release of melatonin after 24 hours. The quantity of melatonin was significantly increased after 2, 5 and 8 hours in comparison to the level 30 minutes before the "day" phase.

In parallel, a blue light stress was induced to another co-culture at the end of each "day" phase in order to reproduce the exposition to digital instruments before sleep. It was found that this condition ("Control Blue Light" in Figure 1) showed a different response: On day 18, i.e. 24 h after the last FCS shock, there was no increase in melatonin release. This difference was statistically significant. These results indicate that the blue light stress disturbed or delayed the cycle of melatonin release.

In the same culture conditions, a third co-culture was treated with Gardenia fruit extract at 0.004% and blue light ("Active 0.004%" in Figure 1). It was found that this treatment led to a very similar melatonin release cycle as in the untreated control.

The results of the melatonin release studies are summarized in the following table:

| **Time (h)** | **Untreated** | | **Control Blue Light** | | **Active 0.004%** | |
|---|---|---|---|---|---|---|
| | **Average (pg/ml)** | **SEM** | **Average (pg/ml)** | **SEM** | **Average (pg/ml)** | **SEM** |
| -0.5 | 10.60 | 0.0000 | 10.60 | 0.0000 | 10.60 | 0.0000 |
| 2 | 10.67 | 0.0333 | 10.70 | 0.0000 | 10.70 | 0.0577 |
| 5 | 10.67 | 0.0333 | 10.67 | 0.0667 | 10.63 | 0.0333 |
| 8 | 10.60 | 0.0000 | 10.67 | 0.0333 | 10.70 | 0.0000 |
| 23.5 | 10.70 | 0.0000 | 10.90 | 0.2082 | 10.63 | 0.0333 |
| 26 | 11.43 | 0.2603 | 10.43 | 0.1333 | 11.50 | 0.1155 |
| 29 | 11.80 | 0.1155 | 10.43 | 0.0882 | 11.80 | 0.2082 |
| 32 | 11.50 | 0.1000 | 10.47 | 0.0667 | 11.47 | 0.3180 |
| 47.5 | 10.57 | 0.3180 | 10.57 | 0.0333 | 10.93 | 0.0882 |
| 50 | 11.03 | 0.1856 | 10.53 | 0.1453 | 11.13 | 0.1202 |
| 53 | 10.80 | 0.1528 | 10.60 | 0.0577 | 11.33 | 0.0333 |
| 56 | 11.00 | 0.1732 | 10.87 | 0.0882 | 11.17 | 0.0333 |

As can be clearly seen, both the untreated condition ("Untreated") and that treated with Gardenia fruit extract at 0.004% and blue light (Active 0.004%") showed a significant increase at 2, 5 and 8 hours after the night, i.e. 26, 29 and 32 hours after the last FCS shock, respectively. On the second day after the treatment (day 19), there is a smaller but still noticeable increase in melatonin.

The samples treated with blue light but not the extract ("Control Blue Light") had a significantly lower melatonin release than both other conditions.

In conclusions, it was found that Gardenia fruit extract at 0.004% is able to protect the cells against the effects of blue light exposure. In particular, it allows for preserving the level melatonin release, as well as its cycle.

### Example 5: Mitochondrial Network Analysis and Cell Spreading (in vitro) - Study 1

The mitochondrial network and cell spreading are both biomarkers for cell ageing.

### Cell Culture and Treatment

Human dermal primary fibroblasts from a 57 years old female donor were thawed and amplified in flasks for a few days in CnT-Prime culture medium dedicated to epithelial cell culture (CellnTEC). 24 h before starting the assay, the cells were divided into 3 groups:
- Group 1 was left untreated;
- Group 2 was treated with 0.002% (w/v) of Gardenia fruit extract (from Yunnan rainbow; diluted in culture medium); and
- Group 3 was treated with 0.004% (w/v) of Gardenia fruit extract (from Yunnan rainbow; diluted in culture medium).

Then, the cells were loaded with the Mitotracker Green dye for 15 min. Cells were washed with PBS, detached and seeded into a CYTOOplate with extra-large Y micropatterns at 2000 cells/well in a 10% serum medium.

1.5 h later, once the cells attached and spread on micropatterns, the medium was replaced by a medium containing less serum, further containing the same concentrations of Gardenia fruit extract as previously applied to each of the groups. The cells were then incubated during 2 h at 37 °C with 5% CO₂.

After 2 h of treatment, cells were irradiated with LEDS (reference Kingbright KA-3529AQB25Z4S) at 447 nm for 1 h at 20 J/cm² corresponding to the dose of 1 month (28 days) of screen exposition at 10 cm distance.

Hoechst was added for 15 minutes in each well to stain nuclei. The medium was renewed to wash off Hoechst and cells were incubated in CnT-prime culture medium with the active.

### Image Acquisition

Live imaging was performed on the Leica microscope. At the end of the live imaging, cells were fixed and F-actin was stained with Phalloidin 555. Images were acquired on the Operetta HCS platform from Perkin Elmer.

### Network Analysis

Once the mitochondrial network was detected, the sum of the length of all the filaments of a single cell network was calculated in order to determine the "Network total length", which is averaged between all single cells from the same well. The mitochondrial network can be divided into groups of filaments that are continuously linked: This basal unit is called a "tree". The number of trees per network, as well as their total length were averaged between all single cells detected in each well.

Each tree is divided into "branches" that are delimited at each end by either a junction or an endpoint. These branches were characterized by measuring their average and maximum lengths in the whole network of each single cell ("average branch length").

### Cell Spreading Analysis

A dedicated image analysis was run in order to detect single cells on micropatterns and to measure their area. Correctly spread cells with an area above 1800 µm² were counted.

### Statistical Analysis

The results were statistically analyzed by ordinary ANOVA with multi-comparative. Significance of results is indicated as p<0.05 with *, p<0.01 with ** and p<0.001 with ***.

### Results: Mitochondrial Network

In the literature, it has been described that blue light exposure can lead to an oxidation of the cells and finally impact the mitochondrial network (Rascalou et al., Mitochondrial damage and cytoskeleton reorganization in human dermal fibroblasts exposed to artificial visible light similar to screen-emitted light, Journal of Dermatological Science, 2018, 91: 195-205). The more fragmented the network is, the more disturbed the cell.

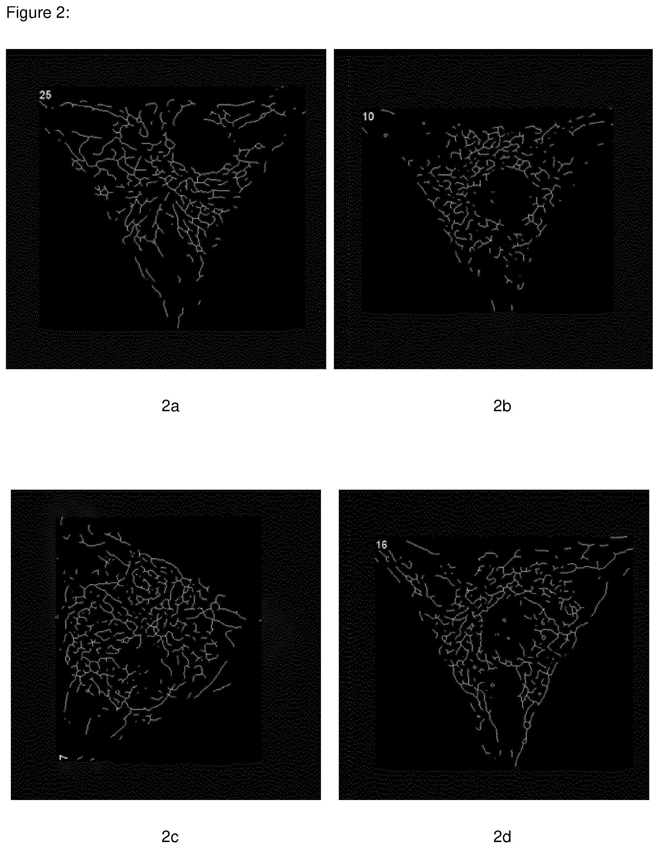

In a first part of the study, the mitochondrial network was analyzed through a network segmentation analysis, the results of which are shown in Figure 2. It was found that the untreated condition showed a clear and defined network of mitochondria (Figure 2a). After exposure to blue light, the network became fragmented and diffused as a result of oxidative stress (Figure 2b). In the presence of Gardenia fruit extract at 0.002%, the network seemed to be less fragmented (Figure 2c). This protective effect was even more pronounced in the presence of Gardenia fruit extract at 0.004% (Figure 2d).

This network was then numerically segmented to perform a quantitative analysis. Various parameters were evaluated, including the total length of the network, the number of trees, the number of branches, and their average lengths.

The results are summarized in the following table:

| | Untreated | Untreated + blue light (20 J/cm²) | Gardenia fruit extract at 0.002% + blue light (20 J/cm²) | Gardenia fruit extract at 0.004% + blue light (20 J/cm²) |
|---|---|---|---|---|
| Network total length | 454.1 ± 21.1 | 259.7 ± 22.0 | 391.9 ± 22.0 | 450.4 ± 20.4 |
| Number of trees | 0.127 ± 0.007 | 0.356 ± 0.015 | 0.223 ± 0.009 | 0.190 ± 0.006 |
| Number of branches | 0.464 ± 0.005 | 0.678 ± 0.012 | 0.549 ± 0.010 | 0.533 ± 0.007 |
| Average tree length | 8.787 ± 0.438 | 2.788 ± 0.114 | 4.476 ± 0.136 | 5.397 ± 0.192 |
| Average branch length | 2.021 ± 0. 025 | 1.334 ± 0.029 | 1.720 ± 0.037 | 1.747 ± 0.025 |

As can be seen from the above, blue light stress led to a significant reduction of the total length of the mitochondrial network. This result confirmed the data of the literature. In presence of the Gardenia fruit extract, however, a significant protection of the network by +68% (p < 0.001) and +98% (p < 0.001), respectively, was observed.

The network total length is automatically linked to the number of trees and branches: if the network is reduced, the number of trees and branches is increased and the average length of each one is decreased.

As expected, blue light stress was found to induce a significant increase of the number of trees and branches, and a significant decrease of the average of tree length and branch length. Again, the Gardenia fruit extracts exhibited a significant protection, reducing the number of trees by 58% and 73% (p < 0.001), and the number of branches by 60% and 68% (p<0.001), respectively.

### Results: Cell Spreading

In second part of the study, the spreading of the cells and their area was analyzed. This spreading is intimately linked to the stress incurred on the cells: when the cell is stressed, its cytoplasm is retracted.

The results are summarized in the following table:

| | Untreated | Untreated + blue light (20 J/cm²) | Gardenia fruit extract at 0.002% + blue light (20 J/cm²) | Gardenia fruit extract at 0.004% + blue light (20 J/cm²) |
|---|---|---|---|---|
| % of spread cells | 69.96 ± 1.83 | 52.10 ± 1.59 | 81.56 ± 1.20 | 78.77 ± 1.03 |
| Average cell area | 2089 ± 20.83 | 1887 ± 20.93 | 2257 ± 20.49 | 2239 ± 21.23 |

It was found that after exposure to blue light, the percentage of spread cells was significantly reduced. The same applies to the average cell area. In presence of Gardenia fruit extract, however, a significant protection was observed, increasing the percentage of spread cells by +165% (p < 0.001) and by +149% (p < 0.001) at 0.002% and at 0.004%, respectively. The cell area was improved by +183% and by +175% at 0.002% and 0.004% of the extract, respectively (p < 0.001).

### Example 6: Mitochondrial Network Analysis (in vitro) - Study 2

The mitochondrial network analysis described in Example 5 was repeated for Gardenia fruit extract at 0.0015% in a natural deep eutectic solvent consisting of 35 wt% of betaine, 40 wt% of glycerol, and 25 wt% of water (NaDES), vs. NaDES alone. The results are summarized in the following table:

| | Untreated | Untreated + blue light (20 J/cm²) | Gardenia fruit extract at 0.0015% in NaDES + blue light (20 J/cm²) | NaDES at 0.0015% + blue light (20 J/cm²) |
|---|---|---|---|---|
| Network total length | 455.31 ± 19.62 | 243.29 ± 12.08 | 297.38 ± 19.15 | 198.88 ± 14.03 |
| Number of trees | 0.15 ± 0.004 | 0.37 ± 0.01 | 0.23 ± 0.02 | 0.37 ± 0.02 |
| Number of branches | 0.48 ± 0.01 | 0.7 ± 0.01 | 0.60 ± 0.01 | 0.69 ± 0.01 |
| Average tree length | 6.69 ± 0.25 | 2.70 ± 0.08 | 4.48 ± 0.46 | 2.80 ± 0.19 |
| Average branch length | 1.92 ± 0.03 | 1.25 ± 0.02 | 1.49 ± 0.06 | 1.27 ± 0.04 |

As can be seen from the above, Gardenia fruit extract in NaDES is able to provide a significant protection of the network: it was able to decrease the tree number by -38% compared to the untreated light stress condition and to increase the average tree length by +66%. The same tendency was observed for the branch parameters: Gardenia fruit extract in NaDES was able to reduce the number of branches by -14% and to increase the average length of the branches by 19%.

The natural deep eutectic solvent alone, on the other hand, has not protective effect against blue light stress.

### Example 7: Protein Oxidation Analysis (ex vivo)

Protein oxidation is another biomarker for cell ageing.

### Culture and Treatments

12 human skin explants of an average diameter of 12 mm (±1 mm) were prepared on an abdominal plasty coming from a 35-year-old Caucasian woman (reference: P2159-AB35, phototype III). The explants were kept in survival in BEM culture medium (BIO-EC's Explants Medium) at 37 °C in a humid, 5%-CO₂ atmosphere.

The explants were assigned to 4 groups as follows (3 explants each):
- Untreated control: explants exposed to light rhythm
- Blue light control: explants exposed to light rhythm and blue light stress
- Gardenia fruit extract (from Indfrag) at 0.002%: explants exposed to light rhythm and blue light stress, with topical application of Gardenia fruit extract at 0.002%
- Gardenia fruit extract at 0.004%: explants exposed to light rhythms and blue light stress, with topical application of Gardenia fruit extract at 0.004%

The Gardenia fruit extracts were prepared by diluting the commercial material (Yunnan Rainbow) in phosphate buffered saline solution (PBS) at the respective concentrations (w/v).

From day 0 to day 4 of the study, skin explants were exposed to a light cycle with the purpose to mimic the circadian rhythm, and to blue light irradiations, according to the following pattern:
- from 7 pm to 7 am (12 hours): exposure of explants to day light using the SlimStyle WO21/02 lamp (Dayvia) which presents an emission spectrum close to solar radiation. Skin explants were kept in BEM culture medium during day light exposure.
- from 7 am to 10 am (3 hours): exposure of explants to a dose of 63.75 J/cm² of blue light, in 1 mL of HBSS medium, using the Solarbox^{®} device (BioEC). The untreated control explants were kept in 1 ml of HBSS, in the dark, during the whole time of blue light exposure. At the end of the exposure, all the explants were put back in 2 mL of BEM medium.
- from 10 am to 7 pm (9 hours): skin explants were kept in the dark, in BEM culture medium.

For the last two groups, Gardenia fruit extract was applied topically on the basis of 2 µl per explant (2 mg/cm²) and spread using a small spatula on days 1, 2, 3 and 4 (before blue light exposure). The untreated control explants did not receive any treatment except the renewal of culture medium.

Half of the culture medium (1.2 ml per well) was renewed on days 1, 2 and 3, at the end of each dark phase of the light cycle.

On day 4, immediately after the last blue light irradiations, 3 explants from each condition were collected and cut into two parts. One part was fixed in buffered formalin and the other part was frozen at -80 °C.

### Immunostaining of Oxidized Proteins

Oxidized proteins were stained on frozen sections after a pre-incubation with DNPH (2,4-dinitrophenylhydrazine, Millipore, ref. 90448) and an incubation with anti-DNP antibody (Millipore, ref. 90451) diluted at 1:250 in PBS, BSA 0.3% for 1 h at 37 °C, with a biotin/streptavidin amplifying system and revealed with VIP, a violet substrate of peroxidase (Vector, ref. SK-4600).

The immunostaining was performed manually and was assessed by microscopic observation.

### Image Analysis: Color Index Quantification

The staining intensities of the oxidized proteins were quantified using two open source optical imaging software programs. Photomicrographs (jpeg format) were opened in GIMP-GNU Image Manipulation Program. The strong-to-light-pink color signals corresponding to the staining were selected, copied and pasted into a new image and saved as a jpeg file, this jpeg file consisting solely of the staining selected. This image was subsequently opened using the ImageJ program. An area within the dermis was selected for analysis. Then, a histogram of the section was created, separating the total number of pixels in the image into 255 color categories spanning the visible spectrum. The peak corresponding to the strong-to-light-pink color was determined by cutting and summing the appropriate counts from each photomicrographs. Alternatively, the numbers corresponding to the peak could be pasted into an Excel spreadsheet and summed.

The pigmentation index was then divided by the surface area (expressed in arbitrary units, A.U.).

### Statistical Analysis

The results were statistically analyzed by Kruskal-Wallis ANOVA followed by Mann Whitney U non-parametric test. Significance of results is indicated as p<0.05 with *, p<0.01 with ** and p<0.001 with ***.

### Results

The results are summarized in the following table:

| | Untreated | Untreated + blue light (20 J/cm²) | Gardenia fruit extract at 0.002% + blue light (20 J/cm²) | Gardenia fruit extract at 0.004% + blue light (20 J/cm²) |
|---|---|---|---|---|
| Oxidized proteins (color index/surface (A.U.)) | 0.010 ± 0.003 | 0.138 ± 0.042 | 0.026 ± 0.003 | 0.019 ± 0.007 |

As can be seen from the above, exposure to blue light led to a significant increase of oxidized proteins (+93%, p < 0.01). In the presence of Gardenia fruit extract, on the other hand, a clear protection from the blue light was observed, demonstrated by a reduction of oxidized proteins by - 81% (p < 0.05) and by -86% (p < 0.01) with the extract at 0.002% and 0.004%, respectively.

### Example 8: Clinical Studies

### Formulation

For the clinical studies described below, a cosmetic formulation having the following INCI formula was used:
AQUA/WATER, CETYL ALCOHOL, GLYCERYL STEARATE, PEG-75 STERATE, CETEH-20, STEARETH-20, ISODECYL NEOPENTANOATE, GARDENIA FRUIT EXTRACT, PHENOXYETHANOL, METHYL PARABEN, PROPYL PARABEN, ETHYL PARABEN, DIMETHICONE, FRAGRANCE, BENZYL SALICYLATE, LINALOOL, D-LIMONENE.

In the placebo composition, the Gardenia fruit extract was omitted.

In more detail:

| INCI | Active | Placebo |
|---|---|---|
| AQUA/WATER | 89.698% (w/v) | 89.700% (w/v) |
| CETYL ALCOHOL, GLYCERYL STEARATE, PEG-75 STEARATE, CETETH-20, STEARETH-20 | 5.0% (w/v) | 5.0% (w/v) |
| ISODECYL NEOPENTANOATE | 4.5% (w/v) | 4.5% (w/v) |
| PHENOXYETHANOL, METHYL PARABEN, PROPYL PARABEN, ETHYL PARABEN | 0.4% (w/v) | 0.4% (w/v) |
| DIMETHICONE | 0.3% (w/v) | 0.3% (w/v) |
| FRAGRANCE, LINALOOL, D-LIMONENE | 0.1% (w/v) | 0.1% (w/v) |
| GARDENIA FRUIT EXTRACT (from Indfrag) | 0.002% (w/v) | -- |

### Panel

The clinical studies were carried out on 40 female volunteers, aged between 18 and 50 with an average age of 35 ± 9 years. Inclusion criteria required the volunteers to have wrinkles on their face and to be in front of a screen (digital devices) at least 4 hours per day, of which 2 consecutive hours during the evening at 100% of the digital devices' luminosity. The volunteers were informed of the possible adverse effects from using the product and the technical conditions, under which the assessment was performed. They willingly signed the consent form which was written in compliance with the Declaration of Helsinki and the December 20th, 1988 act of the Code de la Santé Publique.

During the study, volunteers applied a facial cream containing 0.002% of Gardenia fruit extract (from Indfrag) or a placebo twice daily (morning and evening) for 56 days. The anti-ageing properties of the product was analyzed by the quantification of the number of wrinkles using VISIA^{®} (Canfield) analysis, and the quality of the sleeping cycle was analyzed by a daily log.

### Wrinkle Number Analysis by VISIA^{®}

Using VISIA^{®} (6th generation), digital photographs of the face were obtained on D0, D28, and D56. The control of the repositioning took place directly on the data-processing screen, using an overlay visualization of the images at each time of acquisition. VISIA^{®} allows taking pictures with different types of illuminations and a very rapid capture of images. A series of photos taken under multispectral imaging and analysis allows capturing visual information affecting appearance of the skin.

In this study, the crow's feet wrinkles were analyzed.

### Analysis of Sleep Quality by Daily Log

Volunteers filled in a daily log to collect data on the source of their blue light exposure, the duration of the blue light exposure, tiredness state, easiness to fall asleep, number of nocturnal awakenings, type of skin reactions to the product, and intensity of skin reactions.

### Self-Assessment

The assessment of the sensation felt, efficacy and cosmetic quality of the product was performed through an online questionnaire completed on Eval&Go (https://www.evalandgo.com/) by the volunteers after 27 and 55 days of product application during the study.

### Statistical Analysis

First, the Gaussian law by a Shapiro-Wilk test (α = 0.05) was verified. The data on wrinkles reduction did not follow the Gaussian law; consequently, a non-parametric statistical analysis was done. For the comparison with D0, a paired and non-parametric Wilcoxon test was used (significant result if p < 0.05). Regarding the comparison between the two products (Active and placebo), an unpaired and non-parametric analysis with Mann Whitney test was performed (significant result if p < 0.05).

For the analysis of the self-assessment questionnaire and the daily log results, a Chi-square test was done (dichotomous analysis which consists in comparing the number of associated answers).

### Results: Reduction of Number of Wrinkles

It was found that treatment with the facial cream containing 0.002% of Gardenia fruit extract led to a statistically significant reduction of the number of wrinkles in the crow's feet area by -26% compared to D0.

In addition, it was demonstrated that there was also a statistically significant difference between the facial cream containing 0.002% of Gardenia fruit extract and the placebo of -21% after 56 days of application. In fact, the placebo cream did not have any effect on the wrinkles after 56 days of application.

### Results: Improved Sleep Cycle

During the study, the daily log was used to follow up the number of awakenings during the night and on how easy volunteers fell asleep.

To this end, the questionnaire contained the following three questions, which the volunteers had to answer every day during 56 days:
- Did you wake up during the night? -> YES or NO
- How many times did you wake up?
- Did you fall asleep easily -> YES or NO

After 28 days of application, it was found that only 31.1% of volunteers applying the facial cream containing 0.002% of Gardenia fruit extract had woken up during the night at least once, while 68.9% had never woken up during the night. For the placebo, 49.5% of the volunteers had woken up during the night at least once, while only 50.5% had never woken up during the night. This difference is statistically significant.

After 56 days of application, it was found that, from day 29 to day 56, only 29% of volunteers applying the facial cream containing 0.002% of Gardenia fruit extract had woken up during the night at least once, while 71% had never woken up during the night. For the placebo, 49.6% of the volunteers had woken up during the night at least once, while only 50.4% had never woken up during the night. This difference is again statistically significant.

Thus, it was shown that the Gardenia fruit extract is able to significantly reduce the frequency of awakenings during the night in comparison to placebo.

The second question allowed quantifying the number of nightly awakenings over the study period. It was found that volunteers applying the placebo had, on average, woken up 23 times in the first 28 days and 41.1 times in the total 56 days. Volunteers applying the facial cream containing 0.002% of Gardenia fruit extract, on the other hand, had only woken up 3 times in the first 28 days and 7.5 times in the total 56 days, on average. Thus, the Gardenia fruit extract led to a significant reduction in the number of awakenings by -87% and -82% after 28 and 56 days, respectively, compared to the placebo.

These results demonstrated that the Gardenia fruit extract is able to reduce the number of awakenings during the night, thereby improving the sleep quality.

With the third question, the ease of falling asleep was assessed. Using dichotomy analysis, it was shown that, on average, 90.6% of the volunteers applying the facial cream containing 0.002% of Gardenia fruit extract easily fell asleep, while only 84.8% of the volunteers using the placebo said so after 1 month of application. After 2 months, the respective percentages were 89.8% and 85.8%. All these differences were again significant.

### Data Analysis Based on Age of Volunteers

Results were further analysis based on the age of the volunteers tested. To this end, a Younger Group (age 18-35) and an Older Group (age 35-50) were evaluated separately.

Regarding the reduction of wrinkles, it was found that the Gardenia fruit extract (0.002%) of the present invention was more effective for volunteers of the Older Group than for those of the Younger Group: For the Older Group, a significant reduction by -25% in the number of wrinkles was observed after 2 months of application. This effect was also significant compared to the placebo, which only led to a reduction by -5%.

For the frequency of awakenings per night (first question), the results are shown in the following table:

| | | Days 1-28 | | Days 29-56 | |
|---|---|---|---|---|---|
| | | Woke up at least once | Did not wake up | Woke up at least once | Did not wake up |
| Younger Group | Gardenia fruit extract | 22% | 78% | 19% | 81% |
| | Placebo | 60% | 40% | 58% | 42% |
| Older Group | Gardenia fruit extract | 27% | 63% | 29% | 61% |
| | Placebo | 48% | 52% | 51% | 49% |

As can be seen from the above, the Gardenia fruit extract (0.002%) of the present invention led to a significantly smaller number of volunteers of the Younger Group waking up during the night than the placebo. The Older Group displayed the same tendency, but with a slightly lower efficacy.

For the average number of awakenings during the night (second question) a significant and drastic reduction was observed for the Younger Group, with a reduction of -83% and -82% in comparison to the placebo after 28 and 56 days, respectively. For the Older Group, the same tendency was observed; however, the difference between Gardenia fruit extract and placebo was not significant.

Also with regard to the ease of falling asleep, the Gardenia fruit extract of the present invention was found to be more efficient for the Younger Group than for the Older Group.

## Claims

1. Cosmetic active agent comprising a Gardenia fruit extract and a solvent, wherein the solvent is a eutectic solvent having a pH of at least 5.

2. Cosmetic active agent according to claim 1, wherein the eutectic solvent has a pH of at least 5.5, more preferably of at least 6, and most preferably of at least 7.

3. Cosmetic active agent according to claim 1 or 2, wherein the components of the eutectic solvent are of natural origin.

4. Cosmetic active agent according to any one of claims 1 to 3, wherein the eutectic solvent comprises betaine, glycerol and water.

5. Cosmetic active agent according to claim 4, wherein the eutectic solvent comprises from about 30 to about 40 wt% of betaine, from about 35 to about 45 wt% of glycerol, and from about 20 to about 30 wt% of water, more preferably about 35 wt% of betaine, about 40 wt% of glycerol, and about 25 wt% of water.

6. Cosmetic active agent according to any one of claims 1 to 5, wherein the concentration of the Gardenia fruit extract in the cosmetic active agent is from about 0.01 to about 10 wt%, more preferably from about 0.05 to about 1 wt%, and most preferably about 0.1 wt%.

7. Cosmetic active agent according to any one of claims 1 to 6, wherein the Gardenia fruit extract comprises at least 1 wt% of Crocins, more preferably at least 10 wt% of Crocins, and most preferably at least 25 wt% of Crocins.

8. Cosmetic composition comprising the cosmetic active agent according to any one of claims 1 to 7 and a cosmetically acceptable excipient.

9. Cosmetic composition according to claim 8, which is a skin care composition, and in particular an anti-ageing skin care composition.

10. Method of reducing the signs of ageing in skin, comprising the step of topically applying the cosmetic active agent according to any one of claims 1 to 7 or the cosmetic composition according to claim 8 or 9 to the skin, in particular to facial skin.

11. Method of protecting the skin against oxidative stress, comprising the step of topically applying the cosmetic active agent according to any one of claims 1 to 7 or the cosmetic composition according to claim 8 or 9 to the skin, in particular to facial skin.

12. Method of protecting the skin against the effects of blue light, comprising the step of topically applying the cosmetic active agent according to any one of claims 1 to 7 or the cosmetic composition according to claim 8 or 9 to the skin, in particular to facial skin.

13. Non-therapeutic method of protecting an individual's melatonin cycle, comprising the step of topically applying the cosmetic active agent according to any one of claims 1 to 7 or the cosmetic composition according to claim 8 or 9 to the skin, in particular to facial skin.

14. Non-therapeutic method of improving an individual's sleep, comprising the step of topically applying the cosmetic active agent according to any one of claims 1 to 7 or the cosmetic composition according to claim 8 or 9 to the skin, in particular to facial skin.

15. Use of a Gardenia fruit extract for improving an individual's sleep.

## Patentansprüche

1. Kosmetischer Wirkstoff, umfassend ein Gardenien-Fruchtextrakt und ein Lösungsmittel, wobei es sich bei dem Lösungsmittel um ein eutektisches Lösungsmittel mit einem pH-Wert von mindestens 5 handelt.

2. Kosmetischer Wirkstoff nach Anspruch 1, wobei das eutektische Lösungsmittel einen pH-Wert von mindestens 5,5, bevorzugter von mindestens 6 und höchst bevorzugt von mindestens 7 aufweist.

3. Kosmetischer Wirkstoff nach Anspruch 1 oder 2, wobei die Komponenten des eutektischen Lösungsmittels natürlichen Ursprungs sind.

4. Kosmetischer Wirkstoff nach einem der Ansprüche 1 bis 3, wobei das eutektische Lösungsmittel Betain, Glycerin und Wasser umfasst.

5. Kosmetischer Wirkstoff nach Anspruch 4, wobei das eutektische Lösungsmittel etwa 30 bis etwa 40 Gew.-% Betain, etwa 35 bis etwa 45 Gew.-% Glycerin und etwa 20 bis etwa 30 Gew.-% Wasser, bevorzugter etwa 35 Gew.-% Betain, etwa 40 Gew.-% Glycerin und etwa 25 Gew.-% Wasser umfasst.

6. Kosmetischer Wirkstoff nach einem der Ansprüche 1 bis 5, wobei die Konzentration des Gardenien-Fruchtextrakts in dem kosmetischen Wirkstoff etwa 0,01 bis etwa 10 Gew.-%, bevorzugter etwa 0,05 bis etwa 1 Gew.-% und höchst bevorzugt etwa 0,1 Gew.-% beträgt.

7. Kosmetischer Wirkstoff nach einem der Ansprüche 1 bis 6, wobei das Gardenien-Fruchtextrakt mindestens 1 Gew.-% Crocine, bevorzugter mindestens 10 Gew.-% Crocine und höchst bevorzugt mindestens 25 Gew.-% Crocine umfasst.

8. Kosmetische Zusammensetzung, umfassend den kosmetischen Wirkstoff nach einem der Ansprüche 1 bis 7 und einen kosmetisch unbedenklichen Exzipienten.

9. Kosmetische Zusammensetzung nach Anspruch 8, bei der es sich um eine Hautpflegezusammensetzung und insbesondere eine Anti-Ageing-Hautpflegezusammensetzung handelt.

10. Verfahren zum Verringern der Alterungszeichen der Haut, umfassend den Schritt des topischen Auftragens des kosmetischen Wirkstoffs nach einem der Ansprüche 1 bis 7 oder der kosmetischen Zusammensetzung nach Anspruch 8 oder 9 auf die Haut, insbesondere auf die Gesichtshaut.

11. Verfahren zum Schützen der Haut vor oxidativem Stress, umfassend den Schritt des topischen Auftragens des kosmetischen Wirkstoffs nach einem der Ansprüche 1 bis 7 oder der kosmetischen Zusammensetzung nach Anspruch 8 oder 9 auf die Haut, insbesondere auf die Gesichtshaut.

12. Verfahren zum Schützen der Haut vor den Auswirkungen von blauem Licht, umfassend den Schritt des topischen Auftragens des kosmetischen Wirkstoffs nach einem der Ansprüche 1 bis 7 oder der kosmetischen Zusammensetzung nach Anspruch 8 oder 9 auf die Haut, insbesondere auf die Gesichtshaut.

13. Nicht-therapeutisches Verfahren zum Schützen des Melatoninzyklus eines Individuums, umfassend den Schritt des topischen Auftragens des kosmetischen Wirkstoffs nach einem der Ansprüche 1 bis 7 oder der kosmetischen Zusammensetzung nach Anspruch 8 oder 9 auf die Haut, insbesondere auf die Gesichtshaut.

14. Nicht-therapeutisches Verfahren zum Verbessern des Schlafs eines Individuums, umfassend den Schritt des topischen Auftragens des kosmetischen Wirkstoffs nach einem der Ansprüche 1 bis 7 oder der kosmetischen Zusammensetzung nach Anspruch 8 oder 9 auf die Haut, insbesondere auf die Gesichtshaut.

15. Verwendung eines Gardenien-Fruchtextrakts zum Verbessern des Schlafs eines Individuums.

## Revendications

1. Agent actif cosmétique comprenant un extrait de fruit de Gardenia et un solvant, dans lequel le solvant est un solvant eutectique ayant un pH d'au moins 5.

2. Agent actif cosmétique selon la revendication 1, dans lequel le solvant eutectique a un pH d'au moins 5,5, plus préférablement d'au moins 6, et le plus préférablement d'au moins 7.

3. Agent actif cosmétique selon la revendication 1 ou 2, dans lequel les composants du solvant eutectique sont d'origine naturelle.

4. Agent actif cosmétique selon l'une quelconque des revendications 1 à 3, dans lequel le solvant eutectique comprend de la bétaïne, du glycérol et de l'eau.

5. Agent actif cosmétique selon la revendication 4, dans lequel le solvant eutectique comprend d'environ 30 à environ 40 % en poids de bétaïne, d'environ 35 à environ 45 % en poids de glycérol, et d'environ 20 à environ 30 % en poids d'eau, plus préférablement environ 35 % en poids de bétaïne, environ 40 % en poids de glycérol et environ 25 % en poids d'eau.

6. Agent actif cosmétique selon l'une quelconque des revendications 1 à 5, dans lequel la concentration de l'extrait de fruit de Gardenia dans l'agent actif cosmétique est d'environ 0,01 à environ 10 % en poids, plus préférablement d'environ 0,05 à environ 1 % en poids, et le plus préférablement environ 0,1 % en poids.

7. Agent actif cosmétique selon l'une quelconque des revendications 1 à 6, dans lequel l'extrait de fruit de Gardenia comprend au moins 1 % en poids de Crocines, plus préférablement au moins 10 % en poids de Crocines, et le plus préférablement au moins 25 % en poids de Crocines.

8. Composition cosmétique comprenant l'agent actif cosmétique selon l'une quelconque des revendications 1 à 7 et un excipient cosmétiquement acceptable.

9. Composition cosmétique selon la revendication 8, qui est une composition de soin de la peau, et en particulier une composition de soin de la peau anti-âge.

10. Procédé de diminution des signes du vieillissement cutané, comprenant l'étape d'application par voie topique de l'agent actif cosmétique selon l'une quelconque des revendications 1 à 7 ou de la composition cosmétique selon la revendication 8 ou 9 sur la peau, en particulier sur la peau du visage.

11. Procédé de protection de la peau contre le stress oxydant, comprenant l'étape d'application par voie topique de l'agent actif cosmétique selon l'une quelconque des revendications 1 à 7 ou de la composition cosmétique selon la revendication 8 ou 9 sur la peau, en particulier sur la peau du visage.

12. Procédé pour protéger la peau contre les effets de la lumière bleue, comprenant l'étape d'application par voie topique de l'agent actif cosmétique selon l'une quelconque des revendications 1 à 7 ou de la composition cosmétique selon la revendication 8 ou 9 sur la peau, en particulier sur la peau du visage.

13. Procédé non thérapeutique de protection du cycle de la mélatonine d'un individu, comprenant l'étape d'application par voie topique de l'agent actif cosmétique selon l'une quelconque des revendications 1 à 7 ou de la composition cosmétique selon la revendication 8 ou 9 sur la peau, en particulier sur la peau du visage.

14. Procédé non thérapeutique d'amélioration du sommeil d'un individu comprenant l'étape d'application par voie topique de l'agent actif cosmétique selon l'une quelconque des revendications 1 à 7 ou de la composition cosmétique selon la revendication 8 ou 9 sur la peau, en particulier sur la peau du visage.

15. Utilisation d'un extrait de fruit de Gardenia pour améliorer le sommeil d'un individu.
